# DEMANDE DE BREVET EUROPEEN

(11) **EP 1 264 592 A1**
(43) Date de publication de la demande: **11.12.2002**
(21) Numéro de dépôt: 02291369.3
(22) Date de dépôt: 04.06.2002
(51) Int. Cl.: A61K 7/48, A47K 7/02

(54) **Dispositif pour l'application d'un produit de bronzage artificiel**

(30) Priorité: 05.06.2001 FR 0107309
(71) Demandeur: L'OREAL, 75008 Paris (FR)
(72) Inventeur: Gueret, Jean-Louis, 75016 Paris (FR)
(74) Mandataire: Tanty, François

(57) **Abrégé**

L'invention concerne un dispositif pour l'application d'un produit de bronzage artificiel sur la peau.

Il comporte une matrice adhésive (101) disposée entre au moins deux couches (102, 103) liées de manière permanente à la matrice (101), l'une au moins de ces couches (102, 103) étant perméable à un liquide, et par le fait que la matrice (101) contient au moins un produit de bronzage artificiel apte à exercer son action sur la peau lorsque la matrice (101) est mise au contact dudit liquide.

## Description

La présente invention concerne l'application de produits de bronzage artificiel.

Il est connu de conditionner les crèmes de bronzage artificiel dans des pots ou tubes et de les appliquer avec les doigts sur le corps ou le visage. Il est difficile d'obtenir une application uniforme du produit, ce qui entraîne un risque de coloration non homogène de la peau. De plus, les doigts utilisés pour étaler le produit sont susceptibles d'être tachés par ce dernier. Enfin, pour assurer la conservation du produit, ce dernier contient des conservateurs dont la présence peut causer une irritation de la peau.

On connaît par le brevet US 5 972 360 une lingette imprégnée d'un produit de bronzage artificiel. Une telle lingette permet d'appliquer plus facilement la quantité voulue de produit sur la peau ; cependant, l'utilisateur est toujours susceptible de se tâcher les doigts au contact de la lingette et d'autre part, pour assurer la conservation du produit, la lingette doit être conditionnée dans une pochette étanche et opaque, donc relativement coûteuse. La lingette n'étant pas sèche, des conservateurs sont susceptibles d'être utilisés.

On connaît par la demande internationale WO 01/08658 une serviette de nettoyage, imprégnée de surfactant. Cette serviette comporte deux couches constituées par exemple par des non-tissés. La serviette peut comporter en outre un produit de bronzage artificiel déposé sur l'une des couches puis recouvert par l'autre couche. L'imprégnation de l'une des couches avec le produit de bronzage artificiel peut se faire notamment par pulvérisation. Il existe le risque que le produit imprègne complètement dans son épaisseur l'une des couches. La fabrication de telles lingettes peut s'avérer relativement complexe.

On connaît par la demande de brevet européen EP 0 998 903 un patch comportant d'une part une matrice pouvant contenir un produit de bronzage artificiel et d'autre part un support perméable. Le produit de bronzage artificiel passe en solution lorsque la matrice est amenée au contact d'un liquide adapté. La matrice est recouverte sur une face par une pellicule de protection amovible. L'application d'un produit de bronzage artificiel au moyen d'un patch collé sur la peau n'est pas toujours satisfaisante, car il existe le risque d'une coloration non homogène de la peau, du fait que le patch reste immobile sur la peau pendant une certaine durée.

Il existe un besoin pour bénéficier d'un dispositif permettant une application aisée d'un produit de bronzage artificiel sur la peau et une bonne conservation de ce produit, tout en diminuant le risque pour l'utilisateur de se tacher les doigts. Il existe également un besoin pour pouvoir obtenir si on le souhaite une coloration non homogène de la surface traitée, afin de former un décor par exemple.

L'invention y parvient grâce à un nouveau dispositif d'application, caractérisé par le fait qu'il comporte une matrice disposée entre au moins deux couches liées de manière permanente à la matrice, l'une au moins de ces couches étant perméable à un liquide, la matrice contenant au moins un produit de bronzage artificiel apte à exercer son action sur la peau lorsque la matrice est mise au contact dudit liquide, lequel peut être de l'eau, par exemple.

Par "produit de bronzage artificiel", on englobe au sens de la présente invention les composés qui produisent d'eux-mêmes une coloration sur la peau, à savoir les autobronzants tels que la DHA (dihydroxycétone) ou l'erytrulose, par exemple, et ceux qui nécessitent une exposition aux UV, à savoir les accélérateurs de bronzage, tels que les précurseurs de la mélanine, par exemple. Le produit de bronzage artificiel peut être teinté ou non.

Au sens de la présente, deux éléments structurels ou plus « liés de manière permanente » ne peuvent être aisément séparés l'un de l'autre en appliquant une force de traction exercée de manière complètement manuelle. Par exemple, une pellicule de protection amovible au contact d'un adhésif d'un pansement ne serait pas considérée comme étant liée de manière permanente au reste du bandage.

L'invention permet une bonne conservation du produit de bronzage artificiel, sans recours à des conservateurs, car celui-ci peut être stocké en milieu sensiblement anhydre au sein de la matrice. La fabrication du dispositif est relativement simple, puisque c'est la matrice contenant le produit de bronzage artificiel qui assure la cohésion des couches entre lesquelles elle est située.

De plus, le dispositif peut être réalisé de manière à ne pas être adhésif, afin de pouvoir être facilement déplacé au contact de la peau lors de l'application, ce qui permet d'obtenir une coloration homogène. Le cas échéant, le dispositif peut être réalisé avec un certain contour et la couche perméable tournée vers la peau peut être réalisée de manière à permettre à la matrice, après imprégnation du dispositif par le liquide, d'éclater et de traverser la couche perméable. La matrice rend alors la face extérieure de la couche perméable adhésive, ce qui permet de la maintenir immobile sur la peau pendant une durée suffisante pour réaliser le motif souhaité en changeant la couleur de la peau. En variante, ou additionnellement le cas échéant, la couche perméable peut être rendue imperméable au produit de bronzage artificiel par endroits seulement, par exemple en recouvrant la couche perméable d'un voile imperméable ajouré par endroits ou en réalisant sur la couche perméable une impression sélective, éventuellement tramée, d'une substance gênant, voire empêchant, la diffusion du produit de bronzage artificiel vers la peau, ou au contraire l'accélérant.

Le motif réalisé sur la peau peut être par exemple un dessin de lune, d'étoile, ou tout autre forme.

Dans une réalisation particulière, la matrice contient un ou plusieurs composés solubles dans le liquide utilisé, notamment dans une quantité suffisante pour que la matrice perde de sa cohésion au contact dudit liquide. Le relargage du produit de bronzage artificiel, au travers de la couche perméable tournée vers la peau, peut s'en trouver facilité.

Dans une réalisation particulière, la matrice contient une charge d'un ou plusieurs composés capables de gonfler au contact dudit liquide, notamment dans une quantité suffisante pour que la matrice perde de sa cohésion au contact dudit liquide. Comme précédemment, le relargage du produit de bronzage artificiel peut s'en trouver facilité.

La matrice peut également comporter un ou plusieurs composés absorbeurs d'humidité, notamment comporter entre 0,2 et 60 %, voire entre 0,5 et 40 %, en masse d'un composé absorbeur d'humidité. Ce composé peut être choisi par exemple dans la liste suivante : polyacrylates, silice, fibres de coton, amidons, alginates, carbonates de calcium ou de magnésium, viscose, cellulose, lyophilisats.

La matrice peut encore contenir une charge d'un ou plusieurs composés sensiblement inertes, notamment une poudre de polyamide ou des microbilles, afin par exemple de favoriser son éclatement au contact dudit liquide.

La matrice peut comporter, outre un produit de bronzage artificiel, un ou plusieurs actifs choisis dans la liste suivante : vitamine C, vitamine A, vitamine F, glycérine, laponite, tensioactifs, collagène, acide salicylique, acide tio, caféine, huiles essentielles aromatiques, colorants, anti-oxydants, anti-radicaux libres, hydratants, dépigmentants, liporégulateurs, anti-acnéiques, anti-séborrhéiques, agent anti-vieillissement, adoucissants, antirides, agents kératolitiques, agent anti-inflammatoires, rafraîchissants, cicatrisants, protecteurs vasculaires, anti-bactériens, antifongiques, anti-perspirants, déodorants, conditionneurs de la peau, insensibilisants, immunomodulateurs, agents nourrissants, mélatonine, DHEA.

La matrice peut également comporter des particules aimantables ou aimantées.

La matrice adhésive peut être à base d'un adhésif non soluble dans l'eau à l'état réticulé.

La matrice adhésive peut être à base d'un adhésif permanent choisi dans la liste suivante : adhésifs à base vinylique, à base de PVA ou de PVP, à base de pseudo latex, à base de polymères acryliques, de polyuréthanes ou d'élastomères de latex. L'adhésif sera choisi de manière à être compatible avec le produit de bronzage artificiel et le ou les actifs utilisés, le cas échéant.

La matrice peut être directement liée à une couche choisie parmi les suivantes : un non-tissé, une mousse, un tissé, un film plastique ou métallisé, perforé ou non. Le non-tissé peut être un non-tissé aéré et perforé, afin de permettre à la matrice, après contact avec le liquide utilisé, de passer après éclatement à travers les perforations du non-tissé et entre les fibres hors des perforations, le cas échéant, ce qui facilite le transfert du produit de bronzage artificiel vers la peau et peut conférer à la face extérieure concernée des propriétés adhésives.

Dans une réalisation particulière, la matrice est disposée entre une couche perméable et une couche imperméable audit liquide. La matrice peut notamment être disposée entre une couche hydrophile et une couche imperméable audit liquide, par exemple entre un non-tissé hydrophile et un film plastique imperméable. Le film plastique étanche peut être lui-même recouvert d'un non-tissé ou d'un flocage. La présence d'une couche imperméable est utile pour isoler les doigts de l'utilisateur de la matrice au moment de l'utilisation, donc d'éviter à l'utilisateur de se tacher les doigts.

Dans une autre réalisation particulière, la matrice est disposée entre deux couches perméables audit liquide. La matrice peut notamment être disposée entre deux couches hydrophiles, ou entre une couche hydrophile et une couche hydrophobe. La matrice peut ainsi être disposée entre d'une part un tissé ou un non-tissé hydrophile et d'autre part un tissé ou non-tissé hydrophobe. En variante, la matrice peut être disposée entre un film plastique perforé et un non-tissé hydrophile. L'utilisation d'un non-tissé hydrophobe permet d'obtenir un mauvais mouillage d'une face du dispositif, destinée à servir de face de préhension, tout en ayant une sensation au toucher plus agréable que celle conférée par un simple film plastique étanche.

Le dispositif peut se présenter par exemple sous la forme d'un disque, d'une serviette ou d'un gant. Dans ce dernier cas, le gant comporte avantageusement une couche imperméable au liquide utilisé, entre la matrice et l'intérieur du gant.

Grâce à l'invention, les couches entre lesquelles est disposée la matrice peuvent être dépourvues sur leurs faces opposées à la matrice du produit de bronzage artificiel, avant la mise au contact de la matrice avec ledit liquide. Ainsi, le produit de bronzage artificiel est relativement bien protégé des rayons UV notamment, en particulier lorsque les couches extérieures à la matrice sont aptes à constituer une barrière vis-à-vis du rayonnement UV. Il n'est donc pas nécessaire de prévoir un conditionnement individuel opaque, et le coût du dispositif s'en trouve considérablement réduit.

Selon un autre de ses aspects, l'invention a encore pour objet un article pour appliquer un produit de bronzage sur la peau, comportant
- une première couche,
- une deuxième couche,
- une matrice adhésive disposée entre les première et deuxième couches, cette matrice adhésive comportant un adhésif et un produit de bronzage, article dans lequel après contact avec un liquide, par exemple de l'eau, pour appliquer le produit de bronzage, la matrice adhésive se dégrade au moins partiellement pour libérer le produit de bronzage, la première couche étant perméable au liquide de telle sorte que le produit de bronzage puisse être appliqué sur la peau lorsque l'article est mouillé avec le liquide, la deuxième couche étant imperméable au liquide. Le produit de bronzage peut être soluble dans le liquide.

L'invention a encore pour objet une boîte comportant une pluralité de disques, de serviettes ou de gants conformes à l'invention.

L'invention a encore pour objet un kit comportant un dispositif tel que défini plus haut et un récipient contenant ledit liquide, ce dernier étant par exemple une lotion hydroalcoolique, et pouvant contenir un ou plusieurs actifs susceptibles de favoriser la pénétration dans la peau du produit de bronzage artificiel.

Le dispositif peut se présenter en rouleau, avec des prédécoupes éventuellement pour faciliter la distribution de feuilles.

L'invention a encore pour objet un procédé de fabrication, comportant les étapes suivantes :
(a) incorporer dans une matrice à base d'adhésif un produit de bronzage artificiel,
(b) disposer la matrice entre au moins deux couches, dont l'une au moins est perméable à un liquide, ce liquide permettant au produit de bronzage artificiel, lorsque la matrice est mise au contact dudit liquide, d'exercer son action sur la peau.

La matrice peut ne pas passer par une phase aqueuse lors de la fabrication du dispositif, ce qui permet d'éviter l'emploi de conservateurs.

Dans une mise en oeuvre particulière du procédé, la matrice est assemblée avec l'une des couches sans imprégner cette dernière sur toute son épaisseur, n'imprégnant par exemple que 25% au plus de son épaisseur. L'autre couche, par exemple la couche la plus perméable au liquide utilisé et à travers laquelle le produit de bronzage artificiel sera libéré vers la peau, peut être fixée sur la matrice par simple contact adhésif. La matrice est ainsi éloignée, avant sa mise en contact avec le liquide, des faces extérieures du dispositif, ce qui permet de protéger la matrice, notamment du rayonnement UV.

L'invention sera mieux comprise à la lecture de la description détaillée qui va suivre, d'exemples de mise en oeuvre non limitatifs de l'invention, et à l'examen du dessin annexé (qui fait partie intégrante de la description), sur lequel :
- les figures 1 à 3 représentent divers exemples de dispositifs d'application conformes à l'invention,
- les figures 4 à 10 représentent divers exemples de structures possibles,
- la figure 11 illustre la fabrication de la structure de la figure 10,
- la figure 12 est une section selon le trait de coupe XII-XII de la figure 1,
- la figure 13 représente de manière schématique un dispositif découpé de manière à permettre de réaliser un motif sur la peau,
- la figure 14 représente une variante permettant de réaliser un décor sur la peau,
- la figure 15 est une section schématique selon le trait de coupe XV-XV de la figure 14, et
- la figure 16 représente de manière schématique un non-tissé aéré perforé.

Sur les figures, les proportions relatives des différents éléments n'ont pas toujours été respectées, dans un souci de clarté du dessin.

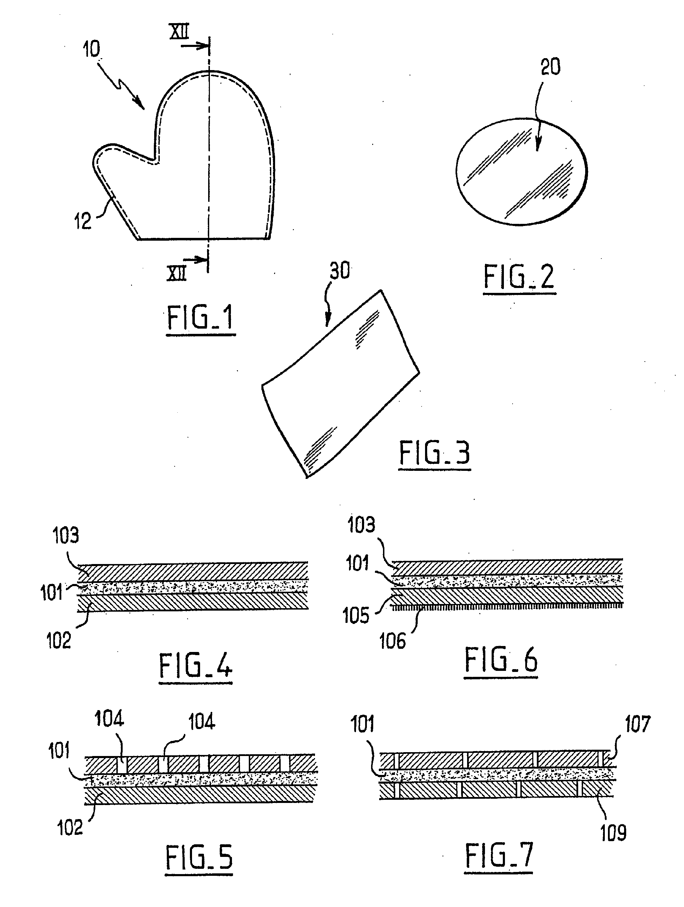

Le dispositif d'application selon l'invention peut présenter des formes diverses, notamment se présenter sous la forme d'un gant 10, comme représenté sur la figure 1, sous la forme d'un disque 20, comme représenté sur la figure 2, ou sous la forme d'une serviette 30, comme représenté sur la figure 3. Dans tous les cas, le dispositif comprend une structure composite dont on va maintenant décrire, en se reportant aux figures 4 à 10, différents exemples, non limitatifs.

D'une manière générale, la structure composite comprend une matrice adhésive qui contient le produit de bronzage artificiel et une couche d'un matériau différent de celui de la matrice de part et d'autre de celle-ci, au moins l'une des couches étant perméable à un liquide permettant de faire passer en solution le produit de bronzage artificiel afin de l'appliquer sur la peau de l'utilisateur.

La structure composite peut se présenter sous la forme d'une feuille relativement souple. Elle peut être utilisée telle quelle ou assemblée avec une autre feuille, pour former un gant par exemple, comme représenté à la figure 1.

Dans l'exemple de la figure 4, la structure composite comporte une matrice adhésive 101 prise en sandwich entre deux couches 102 et 103 perméables au liquide utilisé. La matrice adhésive 101 est à base d'adhésif permanent, non entièrement soluble dans le liquide utilisé, permettant d'assurer une cohésion suffisante des deux couches 102 et 103 même lorsque la structure 100 est mouillée par ledit liquide. Pour constituer les couches 102 et 103, on peut utiliser par exemple un film textile, un non-tissé ou un matériau alvéolaire tel qu'une mousse. La structure composite peut être réalisée en couchant la matrice avec son solvant sur un papier siliconé puis en appliquant un non-tissé sur la matrice. La matrice peut pénétrer sur une faible épaisseur du non-tissé, par exemple moins de 25%. Ensuite, l'ensemble est chauffé pour évaporer le solvant, le papier siliconé est retiré, puis la deuxième couche de non-tissé est appliquée sur la matrice et elle se maintient sur celle-ci par contact adhésif. La deuxième couche peut être constituée par un non-tissé perforé de faible grammage, par exemple. Le procédé de fabrication qui vient d'être décrit permet d'associer à la matrice un tel non-tissé perforé de faible grammage sans craindre que la matière ne l'imprègne sur une grande épaisseur.

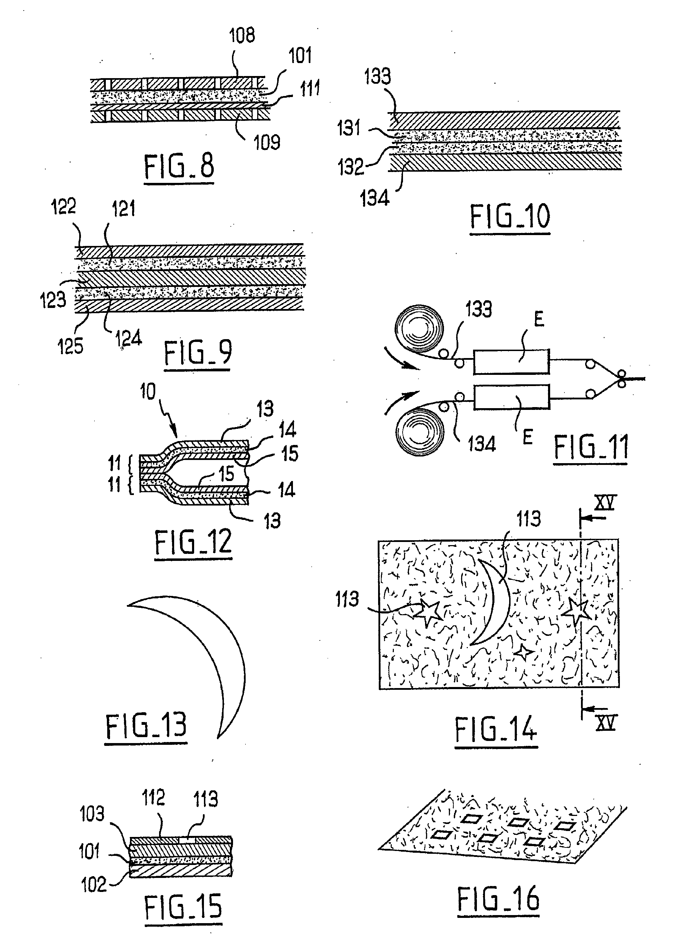

Dans l'exemple de la figure 5, l'une des couches présente des perforations 104 afin de favoriser les échanges entre l'extérieur et la matrice 101. La couche qui comporte ces perforations peut être réalisée dans un matériau perméable, comme la couche 103 de l'exemple précédent ou, en variante, dans un matériau imperméable, par exemple un film plastique. Les perforations peuvent être tournées vers la surface à traiter ou non. On a représenté de façon très schématique à la figure 16 un non-tissé aéré perforé.

On a représenté, sur la figure 6, une structure composite dans laquelle la matrice 101 est disposée entre une couche 103 qui est perméable au liquide utilisé et une couche 105 qui est imperméable à ce liquide et qui est constituée par exemple par un film plastique. Pour améliorer le confort au toucher de ce film plastique, celui-ci peut être revêtu par un flocage 106 ou par un voile d'un non-tissé.

On a illustré sur la figure 7 le fait que la matrice peut être disposée entre deux couches perforées 107 et 109. Le matériau utilisé pour réaliser ces couches 107 et 109 peut être une matière plastique, un tissé ou un non-tissé, par exemple. On peut disposer entre l'une des couches extérieures et la matrice un film plastique étanche 111, comme illustré sur la figure 8. Les couches 108 et 109 peuvent être constituées par des non-tissés hydrophiles.

Dans l'exemple de réalisation de la figure 9, la structure composite comporte une première matrice adhésive 121 prise en sandwich entre des couches 122 et 123 et une deuxième matrice adhésive 124 prise en sandwich entre la couche 123 et une autre couche 125. On peut choisir la couche 123, prise en sandwich entre les matrices 121 et 124, dans un matériau perméable au liquide utilisé ou non. Dans le cas où le matériau choisi est imperméable audit liquide, les couches 122 et 125 sont perméables à ce liquide, ce qui permet à ce dernier d'atteindre les matrices 121 et 124 lorsque la structure composite est mouillée. Les matrices 121 et 124 peuvent comporter des actifs différents, ce qui permet à l'utilisateur de traiter la peau de manière différente, selon qu'il choisit d'appliquer sur celle-ci la couche 122 ou la couche 125. Seule la couche 122 permet aux actifs contenus dans la matrice 121 de diffuser vers la peau, compte tenu de l'existence de la barrière constituée par la couche 123. De même, seuls diffusent dans la couche 125 les actifs contenus dans la matrice 124.

On a représenté sur la figure 10 une structure composite comprenant deux matrices 131 et 132 contrecollées, prises en sandwich entre deux couches 133 et 134. L'une de ces couches 133 ou 134 peut être imperméable au liquide utilisé. Les matrices 131 et 132 peuvent contenir par exemple des actifs différents, notamment des actifs ne pouvant pas être conditionnés ensemble.

Pour réaliser la structure représentée à la figure 10, on peut partir, comme illustré sur la figure 11, de couches de support 133 et 134 sur lesquelles on dépose, à des postes d'enduction E connus en eux-mêmes, les matrices 131 et 132. Ces dernières peuvent contenir des solvants, afin de faciliter l'opération d'enduction. Ces solvants peuvent être volatils et éliminés de la structure composite finale par chauffage par exemple. Les couches de support 133 et 134 ainsi revêtues de leurs matrices adhésives respectives sont ensuite contrecollées pour former la structure composite représentée à la figure 10.

On a représenté partiellement sur la figure 12 le gant de la figure 1, en coupe, pour faire apparaître sa structure.

Le gant 10 est formé par l'assemblage de deux feuilles 11 ayant chacune une structure composite, selon une ligne d'assemblage périphérique 12, matérialisée par des pointillés sur la figure 1. L'assemblage peut se faire par thermosondage, par exemple.

Chaque feuille 11 est formée par la superposition de trois couches, à savoir une couche extérieure 13, une matrice 14 et une couche intérieure 15. La matrice 14 adhère aux couches 13 et 15. La couche intérieure 15 est constituée dans l'exemple décrit par un film imperméable, ce qui permet d'isoler la main de l'utilisateur de la matrice 14 lors de l'utilisation. La couche extérieure 13 est formée, dans l'exemple décrit, par non-tissé hydrophile, perméable à un liquide tel que de l'eau ou une solution hydroalcoolique, ce qui permet à l'utilisateur d'amener ce liquide au contact de la matrice 14. Cette dernière renferme un produit de bronzage artificiel et elle est agencée pour libérer ce produit lorsqu'elle est amenée au contact dudit liquide. La composition de la matrice, lorsqu'elle est en phase solvant, est par exemple la suivante (en poids) : 6% polyacrylate, 15% Orgasol®, 5% DHA, 15% glycérine, 2% DHEA, adhésif acrylique en solution dans l'acétate d'éthyle (40% d'extrait sec) qsp 100%. La couche intérieure 15 est réalisée dans un matériau qui permet un assemblage par soudure des deux feuilles 11, par exemple un film PE.

Un dispositif d'application selon l'invention peut être aisément proposé sans conditionnement individuel étanche et opaque, par exemple dans une boîte en carton. Au moment de d'application, on imprègne celui-ci avec un liquide approprié, par exemple de l'eau, de manière à mettre la matrice au contact de ce liquide et provoquer la libération du produit de bronzage artificiel.

Ensuite, le dispositif d'application peut être déplacé sur la peau de manière à permettre au produit de bronzage artificiel libéré par la matrice d'agir sur la peau.

La matrice peut comporter des éléments facilitant la libération du produit de bronzage artificiel et du ou des autres actifs contenus dans la matrice. Ces éléments sont constitués par exemple par des particules hydroabsorbantes ou d'autres charges, par exemple des particules de PE ou de Rilsan® et favorisent l'éclatement de la matrice en créant des interstices qui facilitent le cheminement du liquide et le passage de la matrice à travers les pores ou les perforations de la couche perméable. Il en résulte que la matrice peut conférer en cours d'utilisation des propriétés adhésives à au moins une face de la structure composite. Ces propriétés adhésives peuvent servir par exemple à immobiliser la structure sur la peau afin de réaliser sur celle-ci un décor, lorsque la structure présente un contour particulier, par exemple une forme de lune comme illustré à la figure 13.

La structure peut également comporter des zones de passage préférentiel entre la peau et la matrice, par exemple grâce à un voile 112 pourvu d'ajours 113 correspondants aux motifs à réaliser. Le voile 112 peut être imperméable ou hydrophobe ailleurs qu'au niveau des ajours 113.

En variante, le voile 112 est absent et la couche perméable 103 peut recevoir un traitement, notamment une impression sélective par sérigraphie par exemple afin de colmater, autour des motifs à réaliser, les pores de la couche perméable ou au contraire favoriser, au niveau des motifs, la diffusion du produit de bronzage artificiel vers la peau.

L'invention permet d'éviter l'utilisation de conservateurs puisque ceux-ci ne sont pas indispensables, étant donné que le produit de bronzage artificiel peut être contenu dans une matrice sensiblement anhydre.

De plus, notamment lorsque le dispositif d'application comporte une couche imperméable entre la matrice et la face du dispositif saisie par les doigts de l'utilisateur, celui-ci peut éviter de se tacher les doigts.

Le produit de bronzage artificiel est relativement bien protégé dans la matrice des rayonnements UV extérieurs, ce qui permet d'éviter d'avoir à le conserver dans une pochette opaque.

L'application du produit de bronzage artificiel peut s'effectuer sans crainte d'appliquer celui-ci en excès, du fait de la libération relativement progressive par la matrice de celui-ci.

## Revendications

1. Dispositif (10 ; 20 ; 30) pour l'application d'un produit de bronzage artificiel sur la peau, **caractérisé par le fait qu'**il comporte une matrice adhésive (101) disposée entre au moins deux couches (102, 103) liées de manière permanente à la matrice (101), l'une au moins de ces couches (102, 103) étant perméable à un liquide, et **par le fait que** la matrice (101) contient au moins un produit de bronzage artificiel apte à exercer son action sur la peau lorsque la matrice (101) est mise au contact dudit liquide.

2. Dispositif selon la revendication 1, **caractérisé par le fait que** la matrice (101) contient un ou plusieurs composés solubles dans ledit liquide.

3. Dispositif selon la revendication 1 ou 2, **caractérisé par le fait que** la matrice (101) contient une charge d'un ou plusieurs composés capables de gonfler au contact dudit liquide.

4. Dispositif selon l'une quelconque des revendications 1 à 3, **caractérisé par le fait que** la matrice (101) contient une charge d'un ou plusieurs composés sensiblement inertes.

5. Dispositif selon l'une quelconque des revendications précédentes, **caractérisé par le fait que** ledit liquide est de l'eau ou une solution hydroalcoolique.

6. Dispositif selon l'une quelconque des revendications précédentes, **caractérisé par le fait que** la matrice (101) comporte un ou plusieurs composés absorbeurs d'humidité.

7. Dispositif selon la revendication précédente, **caractérisé par le fait que** la matrice (101) contient entre 0,2 et 60%, voire entre 0,5 et 40%, en masse d'un composé absorbeur d'humidité.

8. Dispositif selon l'une quelconque des deux revendications immédiatement précédentes, **caractérisé par le fait que** la matrice (101) comporte au moins un composé absorbeur d'humidité choisi dans la liste suivante : polyacrylates, silice, fibres de coton, amidons, alginates, carbonates de calcium ou de magnésium, viscose, cellulose, lyophilisats.

9. Dispositif selon l'une quelconque des revendications précédentes, **caractérisé par le fait que** la matrice (101) comporte un ou plusieurs actifs choisis dans la liste suivante : vitamine C, vitamine A, vitamine F, glycérine, laponite, tensioactifs, collagène, acide salicylique, acide tio, caféine, huiles essentielles aromatiques, colorants, anti-oxydants, anti-radicaux libres, hydratants, dépigmentants, liporégulateurs, anti-acnéiques, anti-séborrhéiques, agent anti-vieillissement, adoucissants, antirides, agents kératolitiques, agent anti-inflammatoires, rafraîchissants, cicatrisants, protecteurs vasculaires, anti-bactériens, antifongiques, anti-perspirants, déodorants, conditionneurs de la peau, insensibilisants, immunomodulateurs, agent nourrissants, DHEA, mélatonine.

10. Dispositif selon l'une quelconque des revendications précédentes, **caractérisé par le fait que** la matrice (101) comporte des particules aimantables ou aimantées.

11. Dispositif selon l'une quelconque des revendications précédentes, **caractérisé par le fait que** la matrice (101) est à base d'un adhésif non soluble dans l'eau, à l'état réticulé.

12. Dispositif selon l'une quelconque des revendications précédentes, **caractérisé par le fait que** la matrice (101) est à base d'un adhésif choisi dans la liste suivante : adhésifs à base vinylique, à base de PVA ou de PVP, à base de pseudo latex, à base de polymères acryliques, de polyuréthanes ou d'élastomères de latex.

13. Dispositif selon l'une quelconque des revendications précédentes, **caractérisé par le fait que** le produit de bronzage artificiel est choisi dans la liste suivante : produits autobronzants, accélérateurs de bronzage ; DHA, erythrulose, précurseurs de la mélanine.

14. Dispositif selon l'une quelconque des revendications précédentes, **caractérisé par le fait que** le produit de bronzage artificiel est teinté.

15. Dispositif selon l'une quelconque des revendications précédentes, **caractérisé par le fait que** la matrice (101) est directement liée à au moins une couche choisie parmi les suivantes : un non-tissé, notamment un non-tissé aéré, éventuellement perforé, une mousse, un tissé, un film plastique ou métallisé perforé ou non.

16. Dispositif selon l'une quelconque des revendications précédentes, **caractérisé par le fait que** la matrice (101) est disposée entre une couche perméable (13) et une couche imperméable (15) audit liquide.

17. Dispositif selon l'une quelconque des revendications 1 à 15, **caractérisé par le fait que** la matrice (101) est disposée entre deux couches (102, 103) perméables audit liquide.

18. Dispositif selon l'une quelconque des revendications 1 à 15, **caractérisé par le fait que** la matrice (101) est disposée entre une couche hydrophile et une couche hydrophobe.

19. Dispositif selon l'une quelconque des revendications 1 à 15, **caractérisé par le fait que** la matrice (101) est disposée entre deux couches (102, 103) hydrophiles.

20. Dispositif selon l'une quelconque des revendications 1 à 15, **caractérisé par le fait que** la matrice (101) est disposée entre une couche hydrophile et une couche imperméable audit liquide.

21. Dispositif selon la revendication précédente, **caractérisé par le fait que** la matrice (101) est disposée entre un non-tissé hydrophile et un film plastique imperméable.

22. Dispositif selon l'une quelconque des revendications 1 à 15, **caractérisé par le fait que** la matrice (101) est disposée entre d'une part un tissé ou un non-tissé hydrophile et d'autre part un tissé ou non-tissé hydrophobe.

23. Dispositif selon l'une quelconque des revendications 1 à 15, **caractérisé par le fait que** la matrice (101) est disposée entre un film plastique perforé et un non-tissé hydrophile.

24. Dispositif selon l'une quelconque des revendications 1 à 15, **caractérisé par le fait que** la matrice (101) est disposée entre un film plastique étanche (105) et un non-tissé hydrophile (103) et **par le fait que** le film plastique étanche (105) est lui-même recouvert d'un non-tissé ou d'un flocage (106).

25. Dispositif selon l'une quelconque des revendications précédentes, **caractérisé par le fait que** la matrice (101) est au contact d'un non-tissé aéré perforé.

26. Dispositif selon l'une quelconque des revendications précédentes, **caractérisé par le fait qu'**il se présente sous la forme d'un disque (20).

27. Dispositif selon l'une quelconque des revendications 1 à 25, **caractérisé par le fait qu'**il se présente sous la forme d'une serviette (30).

28. Dispositif selon l'une quelconque des revendications 1 à 25, **caractérisé par le fait qu'**il se présente sous la forme d'un gant (10).

29. Dispositif selon l'une quelconque des revendications 1 à 25, **caractérisé par le fait qu'**il se présente en rouleau, avec éventuellement des prédécoupages.

30. Dispositif selon la revendication 28, **caractérisé par le fait que** le gant comporte une couche (15) imperméable audit liquide entre la matrice (14) et l'intérieur du gant.

31. Dispositif selon l'une quelconque des revendications précédentes, **caractérisé par le fait que** les couches (102, 103) entre lesquelles est disposée la matrice (101) sont dépourvues sur leurs faces opposées à la matrice (101) du produit de bronzage artificiel, avant la mise au contact de la matrice (101) avec ledit liquide.

32. Dispositif selon l'une quelconque des revendications précédentes, **caractérisé par le fait que** lesdites couches (102, 103) sont aptes à constituer une barrière vis-à-vis du rayonnement UV.

33. Dispositif selon l'une quelconque des revendications précédentes, **caractérisé par le fait qu'**il présente la forme d'un motif à réaliser sur la peau.

34. Dispositif selon l'une quelconque des revendications précédentes, **caractérisé par le fait que** la face à appliquer sur la peau présente une perméabilité sélective au produit de bronzage artificiel de manière à réaliser un décor prédéfini sur la peau.

35. Kit comportant un dispositif tel que défini dans l'une quelconque des revendications précédentes et un récipient contenant ledit liquide.

36. Procédé de fabrication d'un dispositif tel que défini dans l'une quelconque des revendications 1 à 34, **caractérisé par le fait qu'**il comporte les étapes suivantes :
a) incorporer dans une matrice (101) à base d'adhésif un produit de bronzage artificiel,
b) disposer la matrice (101) entre au moins deux couches (102, 103), dont l'une au moins est perméable à un liquide, ce liquide permettant, lorsque la matrice (101) est mise à son contact, au produit de bronzage artificiel d'exercer son action sur la peau.

37. Procédé selon la revendication 36, **caractérisé par le fait que** la matrice (101) est assemblée avec l'une des couches (102, 103) sans imprégner cette dernière sur plus de 25% de son épaisseur.

38. Procédé selon la revendication 36 ou 37, **caractérisé par le fait que** la matrice (101) ne passe pas par une phase aqueuse lors de la fabrication.

39. Procédé selon l'une quelconque des revendications 36 à 38, **caractérisé par le fait que** la couche à travers laquelle le produit de bronzage artificiel est libéré vers la peau est fixée sur la matrice (101) par simple contact adhésif.

40. Procédé selon l'une quelconque des revendications 36 à 39, **caractérisé par le fait que** la structure formée par la matrice (101) et les couches (102, 103) disposées de part et d'autre de celle-ci est découpée selon un motif à réaliser sur la peau.

41. Procédé selon l'une quelconque des revendications 36 à 40, **caractérisé par le fait que** la structure formée par la matrice (101) et les couches (102, 103) disposées de part et d'autre de celle-ci est recouverte par un voile ajouré imperméable au produit de bronzage artificiel, de manière à pouvoir réaliser des motifs sur la peau.

42. Procédé selon l'une quelconque des revendications 36 à 41, **caractérisé par le fait qu'**une face du dispositif est traitée de manière sélective de manière à permettre la formation d'un décor sur la peau.
